# FASCICULE DE BREVET EUROPEEN

(11) **EP 0 649 635 B1**
(45) Date de publication et mention de la délivrance du brevet: **16.02.2000**
(21) Numéro de dépôt: 94420287.8
(22) Date de dépôt: 24.10.1994
(51) Int. Cl.: A61B 17/58

(54) **Dispositif de synthèse des fractures de l'extrémité supérieure du fémur**
Vorrichtung zur Zusammenfügung von Knochenbrüchen im oberen Femurende
Device for joining together fractures of the upper extremity of the femur

(30) Priorité: 25.10.1993 FR 9312899
(43) Date de publication de la demande: 26.04.1995
(73) Titulaire: TORNIER SA, 38330 Saint-Ismier (FR)
(72) Inventeur: Judet, Thierry, F-92410 Ville d'Avray (FR); Saillant, Gérard, F-78170 Celle Saint-Cloud (FR)
(74) Mandataire: Karmin, Roger

(56) Documents cités:
- EP-A- 0 515 828
- WO-A-81/02388
- DE-U- 9 303 830
- FR-A- 2 289 154
- US-A- 2 772 676
- US-A- 3 824 995

## Description

La présente invention a trait à un dispositif pour la stabilisation des fractures de l'extrémité supérieure du fémur.

On connaît, par example de EP-A-0 515 828, des dispositifs de ce genre qui comprennent généralement une plaque qui est vissée dans la partie supérieure du fémur. Cette plaque comprend un canon lisse et cylindrique qui est introduit à l'intérieur d'un alésage préalablement ménagé dans la partie interne du fémur. Le canon permet la mise en place d'une vis de traction qui est mise sous tension pour rapprocher la boule céphalique du corps du fémur.

Ces dispositifs comportent une trop grande fragilité et une prise céphalique insuffisante entraînant des risques de rotation axiale de la boule céphalique ou tête fémorale. En outre, ces dispositifs exposent les patients à des risques de luxations, d'infections ou encore de cotyloïdites.

C'est à ces inconvénients qu'entend plus particulièrement remédier la présente invention.

Le dispositif suivant la présente invention a pour but d'améliorer l'ancrage entre la boule céphalique ou tête fémorale et le fémur en vue d'empêcher toute rotation de ladite boule par rapport au corps du fémur. De plus, la structure de ce dispositif permet la remise en charge rapide des patients, c'est-à-dire que ces derniers peuvent reprendre très vite la position verticale et la marche après l'opération.

Le dispositif suivant la présente invention comprend une plaque qui est vissée dans le fémur et comportant dans sa partie supérieure deux canons parallèles et cylindriques qui sont décalés d'une part vers l'antérieur d'un angle α par rapport au plan vertical passant par le milieu de ladite plaque et d'autre part vers le haut d'un angle β par rapport à l'axe horizontal passant par le milieu de la plaque.

En outre, les canons sont décalés latéralement l'un par rapport à l'autre d'une distance X pour répondre parfaitement à la morphologie du fémur.

Le dessin annexé, donné à titre d'exemple, permettra de mieux comprendre l'invention, les caractéristiques qu'elle présente et les avantages qu'elle est susceptible de procurer :
Fig. 1 est une vue en perspective illustrant la plaque qui forme le dispositif suivant la présente invention.
Fig. 2 est une coupe suivant II-II (fig. 1) montrant le décalage vers l'extérieur des canons.
Fig. 3 est une coupe longitunale représentant la mise en place de la plaque par rapport au fémur en vue de réparer la fracture de la tête fémorale.
Fig. 4 et 5 sont des vues illustrant un crochet trochantérien qui permet l'amélioration de la fixation de la plaque dans le fémur.

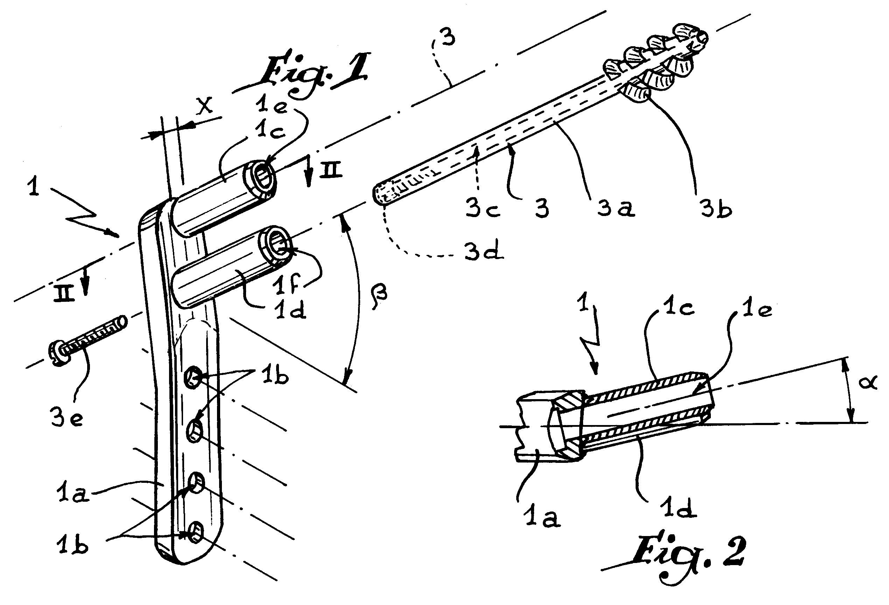

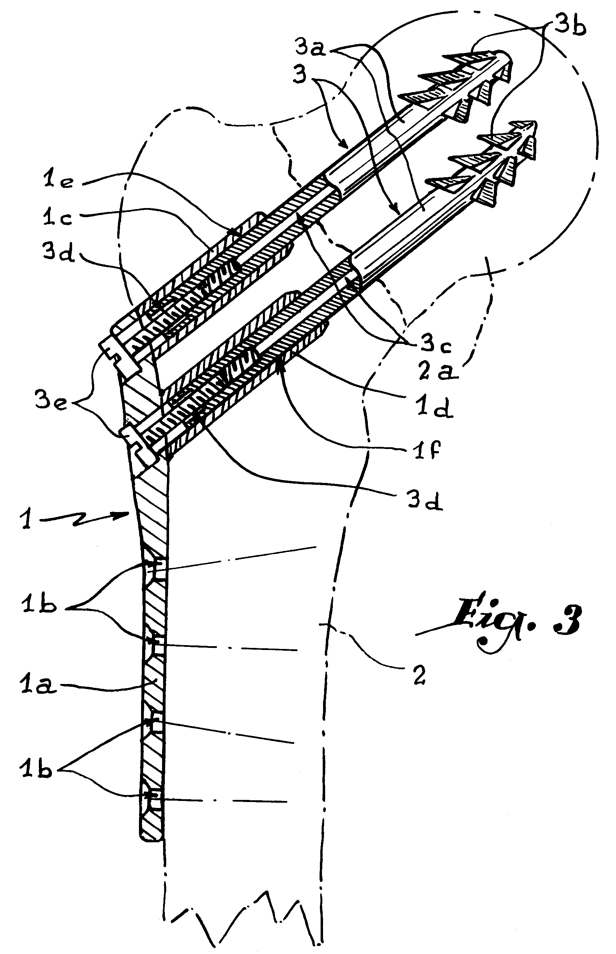

On a représenté en fig. 1 à 3 un dispositif 1 de synthèse des fractures de l'extrémité supérieure d'un fémur 2. Ce dispositif est prévu pour la remise en place de la boule céphalique ou tête fémorale 2a sur le corps du fémur 2.

Le dispositif 1 comprend une plaque 1a dont le profil est adapté à la courbure externe du fémur 2. La partie inférieure de la plaque 1a est percée de plusieurs trous 1b qui permettent sa fixation sur le fémur 2.

La partie supérieure de la plaque 1a est conformée en arc de cercle par rapport à sa partie inférieure de manière à s'adapter parfaitement à la courbure externe du fémur 2 et plus particulièrement au dessous du grand trochanter.

La face dirigée vers le haut de la plaque 1a est solidaire de deux canons cylindriques 1c et 1d dont l'alésage interne est lisse et glissant. Chaque canon 1c, 1d est disposé pour que son alésage interne débouche respectivement dans un alésage épaulé 1e, 1f ménagé dans la partie supérieure de la plaque 1a. L'extrémité libre de chacun des canons 1c et 1d est chamfreinée et arrondie pour faciliter leur mise en place à l'intérieur des trous qui sont préalablement percés dans le fémur 2, comme on le verra mieux plus loin.

Les canons 1c et 1d sont prévus parallèles l'un à l'autre et de longueurs différentes. En effet, le canon 1d est prévu d'une longueur légèrement supérieure à celle du canon 1c.

Les canons 1c et 1d sont décalés vers l'extérieur de la plaque 1a d'un angle α soit à droite, soit à gauche suivant que la stabilisation concerne un fémur droit ou gauche. L'angle α est égal à environ 13° par rapport au plan vertical passant par le milieu de la plaque, de manière à répondre parfaitement à la morphologie du corps fémoral.

Les canons 1c et 1d sont légèrement décalés l'un par rapport à l'autre d'une distance X pour s'adapter à la torsion du col fémoral, c'est-à-dire que le canon inférieur 1d est plutôt plus postérieur par rapport à l'axe central de la plaque 1a, tandis que le canon supérieur 1c est plutôt antérieur par rapport au même axe.

Les canons 1c et 1d sont inclinés d'un angle β par rapport à un axe horizontal passant par le milieu de la plaque 1a. Cet angle β est égal à environ 40°.

La mise en place de la plaque 1a est classique, c'est-à-dire que l'on perce deux trous parallèles dans la partie supérieure du fémur 2 pour la mise en place de deux vis céphaliques 3. Chaque vis céphalique 3 comporte un corps cylindrique 3a dont le diamètre extérieur est ajusté par rapport à celui de l'alésage des canons 1c et 1d de manière à coulisser à l'intérieur de ceux-ci. L'une des extrémités du corps cylindrique 3a comprend un filet 3b à profil conique et auto-taraudant qui vient s'ancrer à l'intérieur de la boule céphalique 2a.

La partie interne du corps cylindrique 3a est percée d'un trou débouchant 3c dont l'extrémité opposée au filet 3b est pourvue d'un alésage à six pans creux 3d permettant de visser en place la vis 3 à l'intérieur de la boule céphalique 2a.

A proximité de l'alésage à six pans creux 3d, le trou 3c est taraudé pour recevoir une vis 3e.

On fixe ensuite la plaque 1a contre le profil extérieur du fémur 2 de manière que les canons 1c pénètrent à l'intérieur de celui-ci en coopérant avec les vis céphaliques 3. Une vis 3e est vissée à l'intérieur de chaque vis céphalique 3 afin de tirer sur celle-ci à l'intérieur des canons 1c et 1d. La mise en place du dispositif 1 permet de plaquer la boule céphalique 2a contre le corps du fémur 2 en vue de la réparation de la fracture.

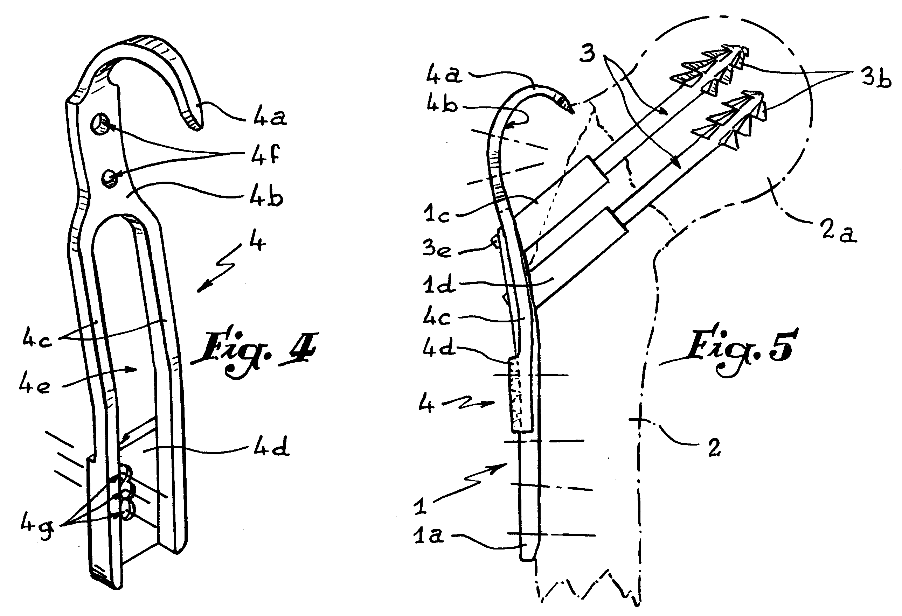

On a représenté en fig. 4 et 5 un élément 4 qui permet la synthèse du massif du grand trochanter lorsque cela le nécessite en plus de la synthèse de la tête et du col du fémur.

L'élément 4 présente dans sa partie supérieure un profil recourbé en forme de crochet 4a simple ou double suivant les conditions d'utilisation. En dessous du crochet 4a est prévue une face d'appui 4b qui se prolonge par deux branches parallèles 4c. Les branches 4c sont réunies l'une à l'autre dans leur partie inférieure par une plaque 4d. Les branches 4c délimitent une lumière 4e qui permet la mise en place de la plaque 1a.

La face d'appui 4b est percée de deux trous 4f permettant la fixation de l'élément 4 dans la partie supérieure du fémur 2 et plus particulièrement sur le grand trochanter. La plaque 4d est percée de plusieurs trous 4g sécants les uns avec les autres pour permettre à une vis de coopérer avec les trous 1b ménagés dans la partie inférieure de la plaque 1a.

L'élément 4 présente un profil adapté à la courbure externe du fémur, et vient se monter en cavalier sur la plaque 1a.

On note que le sommet de la plaque 1a est légèrement affaissé pour ne pas entrer en conflit avec les attaches musculaires situées à cet endroit.

On remarque de plus que plusieurs longueurs de plaques sont disponibles pour répondre à toutes les courbures anatomiques du fémur 2.

On note également que la plaque 1a est réalisée en une matière telle que de l'acier inoxydable ou tout autre matériau bio-combatible permettant d'assurer une fonction et une sécurité parfaites.

## Revendications

1. Dispositif de synthèse des fractures de l'extrémité supérieure du fémur comprenant une plaque qui est vissée dans le fémur et comportant un canon de glissement dans lequel est introduite une vis de mise en tension, caractérisé en ce que la plaque (1a) comporte dans sa partie supérieure deux canons (1c et 1d) parallèles et cylindriques qui sont décalés d'une part vers l'antérieur d'un angles α par rapport au plan vertical passant par le milieu de la plaque (1a) et d'autre part vers le haut d'un angle β par rapport à un axe horizontal passant par le milieu de ladite plaque.

2. Dispositif suivant la revendication 1, caractérisé en ce que les canons (1c et 1d) sont décalés latéralement l'un par rapport à l'autre d'une distance (X) pour répondre parfaitement à la morphologie du fémur (2).

3. Dispositif suivant la revendication 1, caractérisé en ce que la partie supérieure de la plaque (1a) est conformée en arc-de-cercle de manière à s'adapter à la courbure externe du fémur (2).

4. Dispositif suivant la revendication 1, caractérisé en ce que les canons (1c et 1d) comportent des alésages internes lisses pour permettre le coulissement de vis d'ancrage (3) qui sont mises en traction à l'aide de vis (3e) pour plaquer la boule céphalique (2a) sur le fémur (2).

5. Dispositif suivant la revendication 1, caractérisé en ce qu'un élément trochantérien (4) est associé au dispositif (1) par une synthèse complémentaire lors d'une fracture du massif trochantérien.

6. Dispositif suivant la revendication 5, caractérisé en ce que l'élément (4) présente dans sa partie supérieure un profil recourbé en forme de crochet (4a) simple ou double en dessous duquel est prévue une face d'appui (4b) se prolongeant par deux branches parallèles (4c) qui sont réunies à leurs extrémités libres par une plaque (4d).

7. Dispositif suivant la revendication 6, caractérisé en ce que la face d'appui (4b) comporte des trous (4f) pour la fixation de l'élément (4) dans la partie supérieure du fémur (2).

## Claims

1. Device for joining together fractures of the upper end of the femur, comprising a plate which is screwed into the femur and having a sliding barrel into which a tensioning screw is introduced, characterised in that in its upper part the plate (1a) has two parallel, cylindrical barrels (1c and 1d) which are offset, on the one hand towards the front by an angle α with respect to the vertical plane which passes through the middle of the plate (1a), and on the other hand towards the top by an angle β with respect to a horizontal axis which passes through the middle of said plate.

2. Device according to claim 1, characterised in that the barrels (1c and 1d) are offset laterally with respect to one another by a distance (X) so as to perfectly match the morphology of the femur (2).

3. Device according to claim 1, characterised in that the upper part of the plate (1a) is configured as an arc of a circle in a manner whereby it moulds itself to the external curvature of the femur (2).

4. Device according to claim 1, characterised in that the barrels (1c and 1d) have smooth internal bores to permit the sliding of anchoring screws (3) which are placed in traction with the help of screws (3e) in order to plate the cephalic ball (2a) onto the femur (2).

5. Device according to claim 1, characterised in that a trochanteric element (4) is associated with the device (1) by way of a complementary joining together in the event of fracture of the trochanteric body.

6. Device according to claim 5, characterised in that in its upper part the element (4) presents a hook-shaped profile in the shape of a single or double hook (4a), beneath which there is provided a support surface (4b) which continues as two parallel arms (4c) which are joined at their free ends by a plate (4d).

7. Device according to claim 6, characterised in that the support face (4b) has holes (4f) for fixing the element (4) into the upper part of the femur (2).

## Patentansprüche

1. Vorrichtung Zur Zusammenfügung von Knochenbrüchen im oberen Femurende aufgebaut aus einer Platte, die an den Femur anschraubbar ist und einen Gleitzapfen aufweist, in den eine Verspannschraube einsetzbar ist,
dadurch **gekennzeichnet,**
daß die Platte (1a) in ihrem oberen Bereich zwei parallele und zylindrische Zapfen (1c, 1d) aufweist, die einerseits nach vorne gegenüber einer durch die Mitte der Platte (1a) verlaufenden vertikalen Ebene um einen Winkel α und andererseits nach oben gegenüber einer durch die Mitte dieser Platte verlaufenden horizontalen Achse um einen Winkel β versetzt sind.

2. Vorrichtung nach Anspruch 1,
dadurch **gekennzeichnet,**
daß die Zapfen (1c, 1d) um einen Abstand (X) seitlich gegeneinander versetzt sind, um sich der Morphologie des Femurs (2) vollkommen anzupassen.

3. Vorrichtung nach Anspruch 1,
dadurch **gekennzeichnet,**
daß der obere Bereich der Platte (1a) zur Anpassung an die Außenkrümmung des Femurs (2) wie ein Kreisbogen geformt ist.

4. Vorrichtung nach Anspruch 1,
dadurch **gekennzeichnet,**
daß die Zapfen (1c, 1d) glatte Innenbohrungen aufweisen, die die Gleitführung von Ankerschrauben (3) gestatten, die mit Schrauben (3e) angezogen werden, um den Zephalkopf (2a) auf dem Femur (2) aufzusetzen.

5. Vorrichtung nach Anspruch 1,
dadurch **gekennzeichnet,**
daß ein Trochanterelement (4) mit der Vorrichtung (1) über ein entsprechendes Zusammenfügen anläßlich eines Knochenbruches des Trochanterhügels verbunden ist.

6. Vorrichtung nach Anspruch 5,
dadurch **gekennzeichnet,**
daß das Element (4) in seinem oberen Bereich ein vorwärtsgekrümmtes Profil in der Form eines einfachen oder doppelten Hakens (4a) aufweist, unterhalb dem eine Auflagefläche (4b) vorgesehen ist, die sich in zwei parallele Äste (4c) verlängert, die an ihren freien Enden über eine Platte (4d) zusammengeführt sind.

7. Vorrichtung nach Anspruch 6,
dadurch **gekennzeichnet,**
daß die Auflagefläche (4b) Löcher (4f) zur Befestigung des Elements (4) am oberen Abschnitt des Femurs (2) aufweist.
